# EUROPEAN PATENT APPLICATION

(11) **EP 2 455 027 A2**
(43) Date of publication of application: **23.05.2012**
(21) Application number: 11250802.3
(22) Date of filing: 14.09.2011
(51) Int. Cl.: A61B 17/34

(54) **Portal assembly with adjustable height**

(30) Priority: 23.11.2010 US 416529 P; 01.09.2011 US 223335
(71) Applicant: Tyco Healthcare Group LP, New Haven, CT 06511 (US)
(72) Inventor: Davis, Michael, Middletown, CT 06457 (US)
(74) Representative: Soames, Candida Jane

(57) **Abstract**

A portal assembly for positioning within an incision to access an underlying body cavity including a collar and a compressible portal member. The collar includes an elongate member that defines a longitudinal axis and has a longitudinal passageway. The portal member includes one or more longitudinal ports for passage of a surgical object. The portal member is positionable within the longitudinal passageway of the collar to thereby adjust the length of the portal assembly within the incision.

## Description

### CROSS REFERENCE TO RELATED APPLICATION

The present application claims the benefit of and priority to U.S. Provisional Application Serial No. 61/416,529 filed on November 23, 2010, the entire contents of which are incorporated herein by reference.

### BACKGROUND

### Technical Field

The present disclosure relates generally to a portal assembly for use in minimally invasive surgical procedures, such as endoscopic and/or laparoscopic procedures, and more particularly, relates to a portal member and a collar to assist in varying the length of the portal assembly within an incision of a patient.

### Description of Related Art

Minimally invasive surgery is a type of surgery performed through one or more small incisions in a patient's body, usually less than an inch in dimension. Some advantages of minimal invasive surgery is that patients have less trauma to the body, lose less blood, have smaller surgical scars, and need less pain medication.

During a typical minimally invasive procedure, surgical objects, such as surgical access devices, e.g., trocar and cannula assemblies, or endoscopes, are inserted into the patient's body through the incision in tissue. In general, prior to the introduction of the surgical object into the patient's body, insufflation gasses are used to enlarge the area surrounding the target surgical site to create a larger, more accessible work area. Accordingly, the maintenance of a substantially fluid-tight seal is desirable so as to prevent the escape of the insufflation gases and the deflation or collapse of the enlarged surgical site.

To this end, various ports with valves and seals are used during the course of minimally invasive procedures and are widely known in the art. However, a continuing need exists for an access port which can be positioned with bariatric surgery patients.

### SUMMARY

Disclosed herein is a portal assembly for positioning within an incision to access an underlying body cavity including a collar and a compressible portal member and/or surgical port. The collar includes an elongate member that defines a longitudinal axis and has a longitudinal passageway. The portal member includes one or more longitudinal ports for passage of a surgical object. The portal member is positionable within the longitudinal passageway of the collar to thereby adjust the length of the portal assembly within the incision.

In embodiments, the collar includes a flanged portion on a trailing end thereof and a tapered portion on a leading end thereof. The portal member includes a plurality of longitudinal ports. The collar includes a flanged portion on a trailing end of the elongate member.

The flanged portion may include a funnel-shaped configuration to facilitate insertion of the portal member therethrough. The flanged portion includes an underside configured to abut the outer surface of a patient to maintain a proper seal with a body cavity and to prevent the collar from fully entering the incision and the underlying body cavity.

In other embodiments, the collar may include a tapered tip on a leading end of the elongate member whereby the tapered tip has an inner portion. The inner portion of the tapered tip has a first diameter that is smaller diameter than a second diameter of the elongate member. The first and second diameters may range from about 3mm to about 15mm.

In embodiments, a trailing end of the portal member abuts the inner portion of the tapered tip to maintain a seal and a leading end of the portal member abuts a bottom layer of an abdominal wall to maintain a proper seal. The elongate member includes a plurality of inwardly projecting annular ribs disposed along the length thereof in a stacked configuration. The trailing end of the portal member abuts one of the plurality of annular ribs such that an internal sealed relation occurs within the longitudinal passageway. The trailing end of the portal member is configured to abut one of plurality of annular ribs to thereby vary the overall length of portal assembly. The plurality of annular ribs may include a lubricious coating to enhance sliding movement of the portal member therewithin.

These and other features of the current disclosure will be explained in greater detail in the following detailed description of the various embodiments.

### BRIEF DESCRIPTION OF THE DRAWINGS

The above and other aspects, features, and advantages of the present disclosure will become more apparent in light of the following detailed description when taken in conjunction with the accompanying drawings in which:

Fig. 1 is an exploded, perspective view of a surgical port and collar assembly in accordance with the principles of the present disclosure;

Fig. 2 is a perspective, cross-sectional view of an incision made on a tissue layer at a surgical site;

Figs. 3A-3C are a longitudinal, cross-sectional views of the surgical port and collar assembly of FIG. 1A illustrating insertion of the surgical port and collar assembly within the incision at the surgical site; and

Fig. 4 is a longitudinal, cross-sectional view of a surgical port and collar assembly illustrating insertion of the surgical port and collar assembly within the incision at the surgical site in accordance with another embodiment of the present disclosure.

Other features of the present disclosure will become apparent from the following detailed description, taken in conjunction with the accompanying drawings, which illustrate, by way of example, the principles of the present disclosure.

### DETAILED DESCRIPTION

Particular embodiments of the present disclosure will be described herein with reference to the accompanying drawings. As shown in the drawings and as described throughout the following description, and as is traditional when referring to relative positioning on an object, the term "proximal" or "trailing" refers to the end of the apparatus that is closer to the user and the term "distal" or "leading" refers to the end of the apparatus that is further from the user. In the following description, well-known functions or constructions are not described in detail to avoid obscuring the present disclosure in unnecessary detail.

One type of minimal invasive surgery employs an access device that allows multiple instruments to operate through a single entry point, typically the patient's navel. In addition, while certain aspects of this disclosure are described as relating to laparoscopic surgery via the abdominal wall, it should be understood that the present invention is equally relevant to, and may be employed in connection with, other types of surgery such as incision-less surgery, whereby access to a body cavity is provided via a natural orifice such as the vagina, anus, mouth, ear, nasal passage, etc. The disclosed procedure involves insufflating the body cavity and positioning a portal member within, e.g., the navel of the patient. Instruments including an endoscope and additional instruments such as graspers, staplers, forceps or the like may be introduced within the portal member to carry out the surgical procedure.

The portal member used during this surgical procedure may be introduced into an incision (e.g., a Hasson incision) with a Kelly clamp. However, the Kelly clamp may limit the surgeon's ability to properly place a portal member due to the limited length of the Kelly clamp's arm and handle. Furthermore, when performing surgery on an obese patient (e.g., a bariatric related procedure) the length of the portal member may not be adequate enough such that both ends of the portal member engage the abdominal wall to maintain a proper seal. Thus, in accordance with the present disclosure, a collar assembly is used in conjunction with portal member to provide an extension such that proper treatment may be give to an obese patient, as will be described further below.

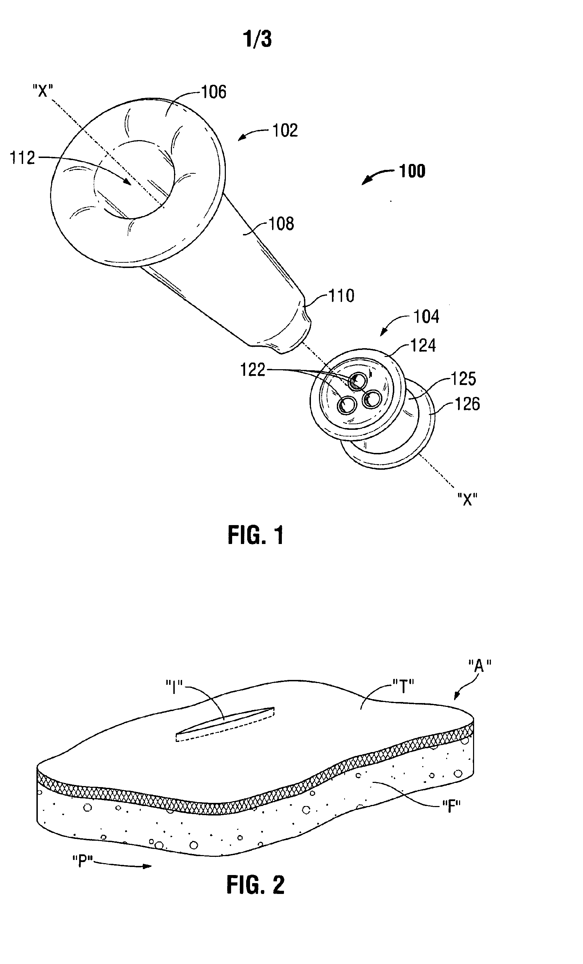

Referring now to the drawings, in which like reference numerals identify identical or substantially similar parts throughout the several views, Figs. 1-4 illustrate a port and collar assembly 100 and 200 for use in a minimally invasive surgical procedure. Surgical port and collar assembly 100 includes a collar 102 and a portal member 104, which is positionable within the collar 102. Collar 102 includes a flanged portion 106 and an elongate member 108 extending therefrom. Flanged portion 106 may have a funnel-shaped configuration to facilitate insertion of portal member 104 therethrough. An underside of flanged portion 106 is configured to abut the outer surface of a patient (e.g., skin) to maintain a proper seal with a body cavity (e.g., peritoneal cavity "P") and to prevent collar 102 from fully entering incision "I."

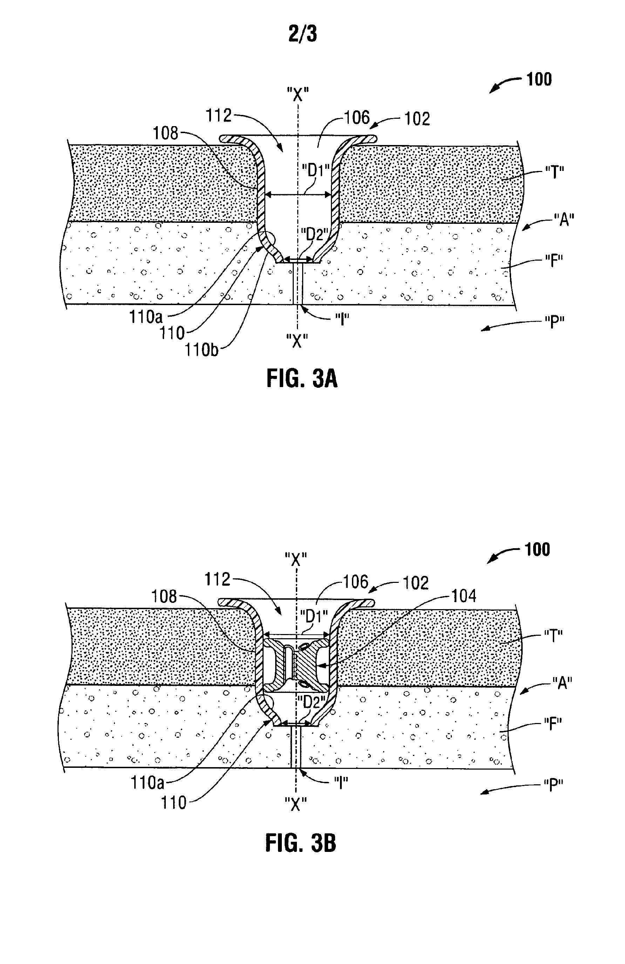

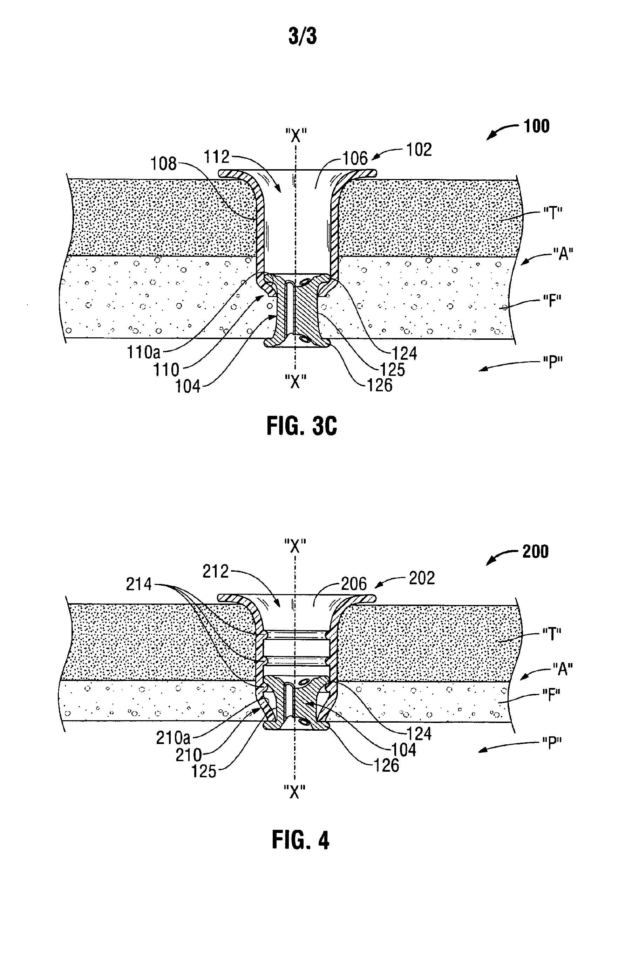

As shown in Figs. 1 and 3A, elongate collar segment or element 108 defines a longitudinal passageway 112 with respect to longitudinal axis "X" extending the length of collar 102. Elongate element 108 is dimensioned for insertion within an incision "I" of an abdominal wall "A" and may be a sleeve element defining an internal diameter "D1." The diameter "D1" may be substantially constant along the length of elongate member 108. Collar 102 includes a tapered tip 110 on a distal end of elongate member 108. As shown in Fig. 3A, tapered tip 110 includes an inner portion 110a and an outer portion 110b, which will be described further below. Diameter "D1" may decrease along the length of elongate member 108 to tapered tip 110 to a diameter "D2." In embodiments, diameters "D1" and "D2" may range from about 3mm to about 15mem.

Portal member 104 includes one or more longitudinal ports 122 that extend along the axis "X" and through elongate portion 125 of the portal member 104. At least one or more inner longitudinal ports 122 are dimensioned to receive a surgical object (not shown) therethrough. Upon introduction through a respective port 122, the inner surface portions defining the port 122 establish and maintain a substantial sealed relation about the instrument or surgical object. Portal member 104 may define an hour glass shape as shown. Trailing and leading ends 124, 126 may define flanged segments which may be integrally formed with elongate portion 125 of portal member 104. Portal member 104 may be made from a disposable, compressible, and/or flexible type material, for example, but not limited to, a suitable foam or gel material having sufficient compliance to form a seal about one or more surgical objects, shown generally as surgical object, and also establish a sealing relation with the tissue. The foam is preferably sufficiently compliant to accommodate off axis motion of the surgical object. In one embodiment, the foam includes a polyisoprene material. Suitable portal members are disclosed in commonly assigned U.S. Patent Application Publication No. 2009/0093752, filed on October 2, 2008, the entire contents of which is hereby incorporated by reference herein.

In operation, incision "I" is made along abdominal wall "A" of a patient to provide access to peritoneal cavity "P" of a patient, as shown in Fig. 2. For purposes of making clear the general nature of the embodiments, a simplified abdominal wall "A" is shown and generally includes a top tissue layer "T" and bottom fat layer "F." Typically, a surgeon performs an incision "I," about 1 inch in length, along the tissue layer "T" and gradually incises every layer, including the fat layer "F", until the peritoneal cavity "P" is accessible. As mentioned above, this procedure is called a Hasson incision.

Referring now to Figs. 2 and 3A-3C, after the proper incisions have been made, collar 102 is positioned (e.g., pushed) into incision "I" in a distal or leading direction within abdominal wall "A" until flanged portion 106 abuts top tissue layer "T" to create a proper seal. After collar 102 is securely positioned within abdominal wall "A," portal member 104 is positioned within flange portion 106 (e.g., within longitudinal passageway 112) and pushed in a distal or leading direction within elongate member 108 until leading end 126 of portal member 104 abuts the bottom layer of abdominal wall "A" (e.g., fat layer "F").

During insertion of portal member 104 within elongate member 108, trailing and leading ends 124, 126 are reduced to an overall width of diameter "D1." During further insertion, leading end 126 of portal member 104 is reduced to a diameter "D2" when pushed or deployed in a distal direction through the opening of tapered tip 110. After further insertion, leading end 126 is pushed through the remaining layers of abdominal wall "A" (e.g., fat layer "F") until the peritoneal cavity "P" is reached. In this configuration, trailing end 124 abuts an inner portion 1 110a of tapered tip 110 to maintain a seal, elongated section 125 is fitted within fat layer "F," and leading end 126 abuts the bottom layer of abdominal wall "A" to maintain a proper seal. That is, portal member 104 prevents the escape of fluids (e.g., gases in a laparoscopic procedure or saline in an arthroscopic procedure) by engaging the internal surface of elongate member 108 and abdominal wall "A," including fat layer "F," in sealed relation therewith. Thereafter, surgical instruments (not shown) may be introduced within one or more longitudinal ports 122 (also in sealed relation as discussed hereinabove) to perform the desired surgery.

Turning now to Fig. 4, which illustrates an alternative embodiment of the present disclosure, collar assembly 200 is depicted and is substantially similar to port and collar assembly 100, described herein above, and will therefore only be described as related to the differences therebetween.

Surgical port and collar assembly 200 includes a collar 202 and a portal member 104 that is positionable within the collar 102. Collar 102 includes a flange portion 206 and an elongate member 108 extending therefrom, which defines a longitudinal passageway 212 with respect to longitudinal axis "X" extending the length of collar 202. Elongate element 208 includes inwardly projecting annular ribs or rings 214 that are disposed along the length thereof in a stacked configuration. Annular ribs 214 provide a surface for proximal or trailing end 124 of portal member 104 to engage therewith. In this manner, when proximal or trailing end 124 abuts any one of annular ribs 214, an internal sealed relation occurs within longitudinal passageway 212. Portal member 104 may be configured to abut any one of annular ribs 214 to thereby vary the overall length of surgical port and collar assembly 200 in its entirety.

Annular ribs 214 may include a lubricious coating such as silicon to enhance sliding movement of portal member. In addition the annular ribs 214 may be, for example, but not limited to, a series of poly (tetrafluoroethylene) (PTFE) lined annular ribs 214 to aid in an effortless deployment of the compressible portal member 104 by permitting the compressible portal member 104 to slide along the annular ribs 214 during advancement or deployment of the compressible portal member 104 through collar 202.

Similar to the surgical procedure described above, after the proper incisions have been made, collar 202 is positioned (e.g., pushed) into incision "I" in a distal or leading direction within abdominal wall "A" until flange portion 206 abuts top tissue layer "T" to create a proper seal. After collar 202 is securely positioned within abdominal wall "A," portal member 104 is positioned within flange portion 206 (e.g., within longitudinal passageway 212) and pushed in a distal or leading direction within elongate member 208 until leading end 126 of portal member 104 abuts the bottom layer of abdominal wall "A" (e.g., fat layer "F").

Depending on the thickness of fat layer "F," during insertion portal member 104 may be adjusted such that trailing end 124 abuts any one of annular ribs 214. In this manner, trailing end 124 of portal member 104 may be adjusted to abut any one of annular ribs 214 to thereby vary the overall length of surgical port and collar assembly 200 in its entirety. In a fully assembled configuration, leading end 126 is flush with the abdominal wall "A" (e.g., fat layer "F"), trailing end 124 abuts one of annular ribs 214 to maintain a seal, and flanged portion 206 abuts top tissue layer "T" to create a proper seal. That is, surgical port and collar assembly 200 prevents the escape of fluids (e.g., gases in a laparoscopic procedure or saline in an arthroscopic procedure) throughout the incision made in the abdominal wall "A," including tissue layer "T" and fat layer "F". Thereafter, surgical instruments (not shown) may be introduced within one or more longitudinal ports 122 (also in sealed relation as discussed hereinabove) to perform the desired surgery.

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

The invention may be described by reference to the following numbered paragraphs:-
1. A portal assembly for positioning within an incision to access an underlying body cavity, the portal assembly comprising: a collar including an elongate member defining a longitudinal axis and having a longitudinal passageway; and a compressible portal member having at least one longitudinal port for passage of a surgical object, wherein the portal member is positionable within the longitudinal passageway of the collar to thereby adjust the length of the portal assembly within the incision.
2. The portal assembly according to paragraph 1, wherein the collar includes a flanged portion on a trailing end thereof and a tapered portion on a leading end thereof.
3. The portal assembly according to paragraph 1, wherein the portal member includes a plurality of longitudinal ports.
4. The portal assembly according to paragraph 1, wherein the collar includes a flanged portion on a trailing end of the elongate member.
5. The portal assembly according to paragraph 4, wherein the flanged portion includes a funnel-shaped configuration to facilitate insertion of the portal member therethrough.
6. The portal assembly according to paragraph 4, wherein the flanged portion includes an underside configured to abut the outer surface of a patient to maintain a proper seal with a body cavity and to prevent the collar from fully entering the incision and the underlying body cavity.
7. The portal assembly according to paragraph 1, wherein the collar includes a tapered tip on a leading end of the elongate member, the tapered tip having an inner portion.
8. The portal assembly according to paragraph 7, wherein the inner portion of the tapered tip has a first diameter that is smaller diameter than a second diameter of the elongate member.
9. The portal assembly according to paragraph 8, wherein the first and second diameters range from about 3mm to about 15mem.
10. The portal assembly according to paragraph 8, wherein a trailing end of the portal member abuts the inner portion of the tapered tip to maintain a seal and a leading end of the portal member abuts a bottom layer of an abdominal wall to maintain a proper seal.
11. The portal assembly according to paragraph 1, wherein the elongate member includes a plurality of inwardly projecting annular ribs disposed along the length thereof in a stacked configuration.
12. The portal assembly according to paragraph 11, wherein the trailing end of the portal member abuts a least one of the plurality of annular ribs such that an internal sealed relation occurs within the longitudinal passageway.
13. The portal assembly according to paragraph 11, wherein the trailing end of the portal member is configured to abut at least one of plurality of annular ribs to thereby vary the overall length of portal assembly.
14. The portal assembly according to paragraph 11, wherein the plurality of annular ribs include a lubricious coating to enhance sliding movement of the portal member therewithin.

## Claims

1. A portal assembly for positioning within an incision to access an underlying body cavity, the portal assembly comprising:
a collar including an elongate member defining a longitudinal axis and having a longitudinal passageway; and
a compressible portal member having at least one longitudinal port for passage of a surgical object, wherein the portal member is positionable within the longitudinal passageway of the collar to thereby adjust the length of the portal assembly within the incision.

2. The portal assembly according to claim 1, wherein the collar includes a flanged portion on a trailing end thereof and a tapered portion on a leading end thereof.

3. The portal assembly according to claim 1 or claim 2, wherein the portal member includes a plurality of longitudinal ports.

4. The portal assembly according to any preceding claim, wherein the collar includes a flanged portion on a trailing end of the elongate member.

5. The portal assembly according to claim 4, wherein the flanged portion includes a funnel-shaped configuration to facilitate insertion of the portal member therethrough.

6. The portal assembly according to claim 4 or claim 5, wherein the flanged portion includes an underside configured to abut the outer surface of a patient to maintain a proper seal with a body cavity and to prevent the collar from fully entering the incision and the underlying body cavity.

7. The portal assembly according to any preceding claim, wherein the collar includes a tapered tip on a leading end of the elongate member, the tapered tip having an inner portion.

8. The portal assembly according to claim 7, wherein the inner portion of the tapered tip has a first diameter that is smaller diameter than a second diameter of the elongate member.

9. The portal assembly according to claim 8, wherein the first and second diameters range from about 3mm to about 15mm.

10. The portal assembly according to claim 8 or claim 9, wherein a trailing end of the portal member abuts the inner portion of the tapered tip to maintain a seal and a leading end of the portal member abuts a bottom layer of an abdominal wall to maintain a proper seal.

11. The portal assembly according to any preceding claim, wherein the elongate member includes a plurality of inwardly projecting annular ribs disposed along the length thereof in a stacked configuration.

12. The portal assembly according to claim 11, wherein the trailing end of the portal member abuts a least one of the plurality of annular ribs such that an internal sealed relation occurs within the longitudinal passageway.

13. The portal assembly according to claim 11 or claim 12, wherein the trailing end of the portal member is configured to abut at least one of plurality of annular ribs to thereby vary the overall length of portal assembly.

14. The portal assembly according to any of claims 11 to 13, wherein the plurality of annular ribs include a lubricious coating to enhance sliding movement of the portal member therewithin.
